# EUROPEAN PATENT SPECIFICATION

(11) **EP 2 052 699 B1**
(45) Date of publication and mention of the grant of the patent: **11.07.2012**
(21) Application number: 06847412.1
(22) Date of filing: 15.11.2006
(51) Int. Cl.: A61F 2/24, C08L 77/00, C08J 5/10, B29C 39/00

(54) **METHOD FOR THE PRODUCTION OF A CUSP FOR ARTIFICIAL CARDIAC VALVE**
VERFAHREN ZUR HERSTELLUNG EINES SEGELS FÜR KÜNSTLICHE HERZKLAPPE
PROCÉDÉ DE FABRICATION DE VOLET POUR VALVE CARDIAQUE ARTIFICIELLE

(30) Priority: 20.07.2006 RU 2006126142
(43) Date of publication of application: 29.04.2009
(73) Proprietor: Samkov, Alexandr Vasilievich, Moscow 117465 (RU)
(72) Inventor: Samkov, Alexandr Vasilievich, Moscow 117465 (RU)
(74) Representative: Andrae, Steffen
(86) International application number: PCT/RU2006/000601
(87) International publication number: WO 2008/010742

(56) References cited:
- EP-A- 0 396 344
- EP-A2- 0 224 153
- WO-A-87/06846
- WO-A-98/40033
- WO-A-2006/125055
- SU-A1- 937 475
- US-A- 3 608 097
- US-A- 5 971 974
- US-A1- 2004 122 514
- US-B1- 6 702 851
- US-B2- 6 908 481
- US-B2- 7 077 801
- LITE POD DAVLENIEM: 'Entsiklopediya polimerov' MOSCOW, SOVETSKAYA ENTSIKLOPEDIYA vol. 2, pages 71 - 84, XP008131164
- 'Khimicheskaya entsiklopediya, Bolshaya Rossiskaya entsiklopediya' MOSCOW 1995, pages 5 - 7

## Description

The invention relates to the medical technology and can be used in manufacturing artificial cardiac valves.

Artificial cardiac valves can comprise one, two, or more cusps having paired protrusions for fastening them in the valve casing. During valve operation, maximal mechanical loading acts onto those protrusions.

Known is a method for manufacturing the cusps of an artificial cardiac valve from a titanium alloy with a coating from pyrocarbon (RU Patent No. 2012284, 1994). However, the cusp from a two-layer material does not possess sufficient reliability when operating continuously for long period of time. Moreover, in mounting the cusp protrusions in casing sockets, metal-to-metal friction problems emerge.

Also known is the method for manufacturing the cusp by obtaining the pyrocarbon layer of controllable thickness on the surface of the graphite substrate by means of: deposing it by decomposing carbon compounds from a gaseous stream in a flow reactor; separating subsequently the pyrocarbon layer from the substrate; and further processing mechanically pyrocarbon in order to shape ift in the form of the cardiac valve cusp (US Patent No. 6,274,191, 2001). The disadvantages of the method are in its long duration and labor intensity, the mechanical processing being performed manually, particularly at the final stage.

Known are methods for manufacturing the valve cusps from materials on a polymer base. According to the SU Author Certificate No. 1144216, 1987, cusps are manufactured from organic-silicon rubber reinforced by threads made from the same material. The RU Patents Nos. 2057494, 1996, and 2153887, 2000, describe methods for manufacturing cusps from polyester urethane. In the first instance, the cusp material includes additionally a reinforcing fabric. In the second instance, the method provides for modifying the cusp surface using the pulse plasma spraying of carbon.

All the described methods are long-term and expensive, and the material of the cusps made by such methods does not keep its mechanical properties and hemocompatibility for a long time, and does not permit for checking the condition of the artificial cardiac valve without surgical intervention.

The claimed invention enables to simplify and cheapen significantly the process for manufacturing the cusps, increase that hemocompatibility and the reliability of the design of the artificial cardiac valve in the most loaded loci, as well as enables to monitor the cusp operation in X-rays.

The method for manufacturing a cusp of an artificial cardiac valve includes steps of: manufacturing a casting mold, and molding subsequently by casting a cusp from a polymer composite comprising 78 to 92 % by weight of polyamide and 8 to 22 % by weight of radiographic contrast medium dispersed therein.

Additionally, the polymer composite can comprise fine acetylene black in amount of 1 to 2 % by weight. The presence of the carbon black in the composite polymer formulation permits it to enhance the tribotechnical characteristics of the artificial cardiac valve, especially in long-term operation thereof.

Preferably barium sulphate (BaSO₄) serves as the radiographic contrast medium. However, this function could be performed by other materials: bismuth oxychloride (BiOCI), bismuth oxide (Bi₂O₃), basic bismuth carbonate (BiO)₂CO₃, etc. In the case, when the composite formulation comprises less than 8 % of the radiographic contrast medium, monitoring the cusp operation in X-rays becomes substantially impossible. When there is more than 22 % of the radiographic contrast medium, the cusp material resistance to alternating loads in the long-term operation decreases to unacceptable levels.

Experience has shown that the polymer composite used for manufacturing the cusps of the artificial cardiac valve can expand during a long-term contact with liquids, particularly blood. In this case, the cusps of the artificial cardiac valve expand in size by 1 to 5 %. Such increase in size could result in disturbances of the cusp operation and even in impaction thereof.

In order to prevent this, the mold for casting the cusp is manufactured for a molding size 1 to 5 % less than necessary, and the cusp is placed after molding into an anticoagulant (e.g., heparin) solution and matured therein until expanding by 1 to 5 %. Thus, a double result is achieved: ensuring the cusp size stability during a long-term operation, and reducing a thrombosis probability in contacting blood with the material of the artificial cardiac valve.

### Examples of the method implementation

Example 1. An initial mixture for manufacturing the cusps of the artificial cardiac valve was prepared for loading into a molding machine. The mixture was prepared by grinding mutually and stirring thoroughly at the same time 86 weight % of polyamide PA12 and 14% by weight of barium sulphate. The resulting mixture was charged into a heated cylinder of the molding machine and heated up to 230 °C. The hot melt was squeezed into the casting mold, whose surface temperature was 75 °C. After 15 s curing, the casting mold was opened, and the formed cusp molding was pushed out onto a soft substrate under room temperature and normal atmospheric pressure. When the casting mold was made thoroughly, the produced cusp required no additional size perfection and surface processing (finishing).

The cusps thus produced (without taking into account the subsequent expansion) are mated for each annular valve casing taking into account a dimensional tolerance in manufacturing thereof, in order to exclude an excessively tight fit of the cusp in the casing and possible impaction of the valve in the case of the cusp expansion.

Example 2. The initial mixture was prepared similarly to the previous example, but with addition of acetylene black. The mixture for the polymer composite comprised 80 % by weight of polyamide PA6 18% by weight of barium sulphate, and 2 % by weight of acetylene black. The casting mold was made for a molding size 2 % less than necessary. The temperature of the mixture prior to squeezing into the casting mold was 240 °C, the temperature of the casting mold surface was 80 °C. After curing, the valve cusp was pushed out of the casting mold into a heparin solution heated to 38 °C and matured therein for 3 hours for expanding. The produced cusp required no additional processing prior to mounting into the casing of the artificial cardiac valve.

Producing the valve cusps by the method of casting from a polymer composite permits to sharply increase the processing rate and reduce the processing cost for manufacturing the artificial cardiac valves.

## Claims

1. A method for manufacturing a cusp having paired protrusions for fastening in a valve casing of an artificial cardiac valve,
including the steps of
- manufacturing a casting mold, the molding size being 1 to 5 % less than necessary for fastening the molded cusp in the valve casing,
- forming a cusp by casting in said casting mold a cusp from a polymer composite comprising 78 to 92 % by weight of polyamide and 8 to 22 % by weight of a radiographic contrast medium dispersed therein, and
- placing the molded cusp after molding into an anticoagulant solution and maturing it therein until an expansion in size by 1 to 5% is obtained.

2. The method according to Claim 1, wherein the polymer composite comprises additionally fine acetylene black in an amount of 1 to 2 % by weight.

3. The method according to Claim 1, wherein the radiographic contrast medium is barium sulphate.

4. The method according to claim 1, wherein the anticoagulant solution is a solution of heparin.

## Patentansprüche

1. Verfahren zur Herstellung einer Ventilklappe mit paarweisen Vorsprüngen zu ihrer Befestigung in einem Ventilgehäuse einer künstlichen Herzklappe,
das die Schritte umfasst
- Herstellen einer Gießform, wobei die Größe des Gussformteils um 1 - 5 % geringer ist als zur Befestigung der geformten Ventilklappe in dem Ventilgehäuse erforderlich ist,
- Bilden einer Ventilklappe durch Gießen einer Ventilklappe in der genannten Gießform aus einem Polymerverbundstoff, der 78 - 92 Gew.-% Polyamid und 8 - 22 Gew.-% eines radiographischen Kontrastmittels, das darin dispergiert ist, umfasst, und
- Anordnen der geformten Ventilklappe nach dem Gießen in einer Lösung eines Antikoagulans und Reifenlassen darin, bis eine Größenexpansion um 1 - 5 % erhalten wurde.

2. Verfahren nach Anspruch 1, wobei der Polymerverbundstoff zusätzlich einen Acetylen-Ruß in einer Menge von 1 - 2 Gew.-% umfasst.

3. Verfahren nach Anspruch 1, wobei das radiographische Kontrastmittel Bariumsulfat ist.

4. Verfahren nach Anspruch 1, wobei die Lösung des Antikoagulans eine Lösung von Heparin ist.

## Revendications

1. Procédé de fabrication d'un volet ayant une paire de projections pour la fixation dans un boîtier de valvule d'une valvule cardiaque artificielle,
incluant les étapes consistant à
- fabriquer un moule de coulée, la taille du moule étant de 1 % à 5 % inférieure à celle qui serait nécessaire pour fixer le volet moulé dans le boîtier de valvule,
- former un volet en coulant un volet dans ledit moule de coulée en un composite polymère comprenant de 78 à 92 % en poids de polyamide et de 8 à 22 % en poids d'un agent de contraste radiographique dispersé dans celui-ci, et
- placer le volet moulé après son moulage dans une solution d'anticoagulant et le laisser mûrir dans cette solution jusqu'à obtenir une expansion en taille de 1 à 5 %.

2. Procédé selon la revendication 1, dans lequel le composite polymère comprend additionnellement du noir d'acétylène fin dans une quantité de 1 à 2 % en poids.

3. Procédé selon la revendication 1, dans lequel l'agent de contraste radiographique est du sulfate de baryum.

4. Procédé selon la revendication 1, dans lequel la solution d'anticoagulant et une solution d'héparine.
